# FASCICULE DE BREVET EUROPEEN

(11) **EP 0 793 721 B1**
(45) Date de publication et mention de la délivrance du brevet: **21.09.2005**
(21) Numéro de dépôt: 95941126.5
(22) Date de dépôt: 22.11.1995
(51) Int. Cl.: C12N 15/12, C07K 14/47, A61K 39/395, C12Q 1/68, A61K 38/17, G01N 33/53

(54) **PEPTIDES CAPABLES DE SE LIER AU DOMAINE SH3 DE LA PROTEINE GAP, SEQUENCES NUCLEOTIDIQUES CODANT POUR CES PEPTIDES, LEUR PREPARATION ET UTILISATION**
PEPTIDE, DIE FÄHIG SIND DIE SH3-DOMÄNE DES GAP-PROTEINS ZU BINDEN, IHRE KODIERENDE DNS, IHRE ZUBEREITUNG UND VERWENDUNG
PEPTIDES CAPABLE OF BINDING TO THE GAP PROTEIN SH3 DOMAIN, NUCLEOTIDE SEQUENCES CODING THEREFOR, AND PREPARATION AND USE THEREOF

(30) Priorité: 22.11.1994 FR 9413955; 16.05.1995 FR 9505753
(43) Date de publication de la demande: 10.09.1997
(73) Titulaire: Aventis Pharma S.A., 92165 Antony Cédex (FR)
(72) Inventeur: DUCHESNE, Marc, F-94370 Sucy-en-Brie (FR); FAUCHER, Didier, F-75013 Paris (FR); PARKER, Fabienne, F-92160 Antony (FR); SCHWEIGHOFFER, Fabien, F-94300 Vincennes (FR); TOCQUE, Bruno, F-92400 Courbevoie (FR)
(74) Mandataire: Le Pennec, Magali
(86) Numéro de dépôt international: PCT/FR1995/001539
(87) Numéro de publication internationale: WO 1996/016169

(56) Documents cités:
- EP-A- 0 496 162
- WO-A-91/02749
- WO-A-94/16069
- FR-A- 2 694 296
- IMMUNOBIOLOGY, vol. 191, no. 2-3, Septembre 1994 pages 128-129, JANSSEN ET AL. 'SH3 binding of GAP-associated p62 : linking protein tyrosine kinases and ras activation in T cells'
- SCIENCE, vol. 259, no. 5094, 22 Janvier 1993 pages 525-528, DUCHESNE ET AL. 'Identification of the SH3 domain of GAP as an essential sequence for ras-GAP mediated signaling'
- EMBO J., vol. 13, no. 6, 15 Mars 1994 pages 1270-1279, YANG ET AL. 'Solution structure of GAP SH3 domain by 1H NMR and spacial arrangement of essential ras signaling involved sequence'
- NATURE, vol. 365, no. 6443, 16 Septembre 1993 pages 269-274, PARK ET AL. 'BUD2 encodes a GTPase-activating protein for Bud1/Rsr1 necessary for proper bud-site selection in yeast'
- J. BIOL. CHEM., vol. 268, no. 35, 15 Décembre 1993 pages 26059-26062, BARFOD ET AL. 'Cloning and expression of a human CDC42 GTPase-activating protein reveals a functional SH3-binding domain'
- MOL. CELL. BIOL., vol. 12, no. 8, Août 1992 pages 3425-3430, MEDEMA ET AL. 'GTPase-activating protein SH2-SH3 domains induce gene expression in a ras-dependent fashion'
- MOL. CELL. BIOL., vol. 12, no. 4, 1992 pages 1835-1845, SEIDEL-DUGAN ET AL. 'Effects of SH2 and SH3 deletions on the functional activities of wild-type and transforming variants of c-src'
- DATABASE STRAND ID= EMST : HSC0FH021; AN: Z42330, 5 Novembre 1994 & C. R. ACAD. SCI. III, SCI. VIE, vol. 318, 1995 pages 263-272, AUFFRAY ET AL. 'IMAGE : intégration au niveau moleculaire de l'analyse du génome humain et de son expression'

## Description

La présente invention concerne de nouvelles séquences peptidiques et nucléotidiques, et leur utilisation pharmaceutique. Plus particulièrement, l'invention concerne des peptides capables de se lier au domaine SH3 de la protéine GAP.

Les produits des gènes ras, généralement désignés protéines p21, jouent un rôle clé dans le contrôle de la division cellulaire chez tous les organismes eucaryotes où ils ont été recherchés. Certaines modifications spécifiques de ces protéines leur font perdre leur contrôle normal et les conduisent à devenir oncogéniques. Ainsi, un grand nombre de tumeurs humaines ont été associées à la présence de gènes ras modifiés. De même, une surexpression de ces protéines p21 peut conduire à un dérèglement de la prolifération cellulaire. Globalement, les protéines p21 sont impliquées dans 30% des cancers humains.
La compréhension du rôle exact de ces protéines p21 constitue donc un des objectifs cibles de la recherche dans le domaine de l'oncologie.
Le modèle dont on dispose actuellement pour expliquer le fonctionnement des protéines p21 repose sur des analogies qu'elles partagent avec les protéines G de transduction. Il existe dans les cellules un équilibre entre les proteines p21 actives, liées à du GTP et les formes inactives, ayant fixé du GDP. Dans une cellule quiescente,
où les p21 ne sont pas sollicitées, la majorité d'entre elles sont sous forme GDP. Lorsque la cellule est stimulée, le facteur d'échange des nucléotides, GEF, devient plus actif et facilite l'évacuation du GDP et son remplacement par du GTP. La protéine adopte alors une conformation active qui lui permet de reconnaître et de stimuler son effecteur, la protéine GAP "GTPase activating protein", vraisemblablement associée à d'autres protéines. Le complexe p21-GTP-GAP interagit vraisemblablement à son tour avec une ou d'autres protéine(s) permettant ainsi la transmission du signal qui entraîne une réponse biologique de la cellule. L'association de p21-GTP avec GAP déclenche simultanément l'hydrolyse du GTP et le retour de la p21 vers la forme inactive.
Dans le cas des protéines p21 oncogènes, la mutation qu'elles portent empêche le retour à l'état inactif. L'équilibre est dans ce dernier cas, déplacé vers la forme active de p21.
Cet équilibre complexe entre les formes active et inactive de p21 est contrôlé à la fois par des facteurs inhérents aux propriétés biochimiques des protéines p21 (affinité relative pour le GDP et le GTP, vitesse d'échange des nucléotides ...) et des facteurs externes modulant leur activité comme notamment la protéine GAP.
La protéine GAP est une protéine cytosolique présente chez tous les organismes eucaryotes qui possède donc la faculté d'accélérer fortement l'hydrolyse du GTP, lié à la protéine p21 normale (Trahey et Mc Cormick 1987). Elle possède deux domaines assurant des fonctions distinctes. Son extrémité carboxy-terminale porte l'activité catalytique qui fixe les protéines p21 et augmente leur activité GTPase. Sur son autre extrémité, en aval de la partie aminoterminale, se trouve une juxtaposition de domaines SH2 et SH3 qui sont susceptibles de participer à des interactions avec d'autres protéines.
A l'heure actuelle, on connait deux protéines interagissant avec la protéine GAP. Il s'agit des protéines dénommées p62 et p190, respectivement de 62kDa et 190kDa. Ces deux protéines étant immunoprécipitées par des anticorps dirigés contre différents épitopes de GAP, forment à l'évidence un complexe spécifique avec GAP. On sait en particulier que c'est au niveau de la région SH2 que se fait l'interaction de la protéine p62 avec la protéine GAP.
En ce qui concerne plus particulièrement le domaine SH3, sa présence dans diverses protéines comme les phospholipases Cγ (PLC-γ), la sous unité p85 de la phophatidyllinositol-3 kinase et la protéine grb-2, toutes impliquées au niveau de la transduction du signal p21-Ras, laisse à penser que ce domaine est tout particulièrement important pour diriger les interactions protéine-protéine et donc nécessaire au fonctionnement de la protéine correspondante et/ou à sa localisation cellulaire. Dans le cas particulier de la protéine GAP, ce domaine SH3 pourrait donc également participer à la transduction du signal Ras. Il est clair que la compréhension du rôle exact de ce domaine SH3 serait particulièrement précieux sur le plan thérapeutique.
La présente invention a précisément pour objectif de contribuer à l'élucidation de la contribution du domaine SH3 à la transduction du signal ras.
La demanderesse a ainsi mis en évidence l'existence de protéines capables de s'associer à la protéine GAP via une liaison directe à son domaine SH3.
plus précisément, la présente invention résulte de l'identification, l'isolement et la caractérisation de protéines capables d'interagir avec le domaine SH3 de la protéine GAP. Elle résulte également de la caractérisation structurale de ces protéines par identification de séquences peptidiques correspondantes.

Plus particulièrement, le polypeptide de l'invention, capable d'interagir avec le domaine SH3 de la protéine GAP, comprend tout ou partie d'une séquence peptidique choisie parmi les séquences SEQ ID N°1, SEQ ID N°2, SEQ ID N°3, SEQ ID N°4, SEQ ID N°5 et SEQ ID N°9.

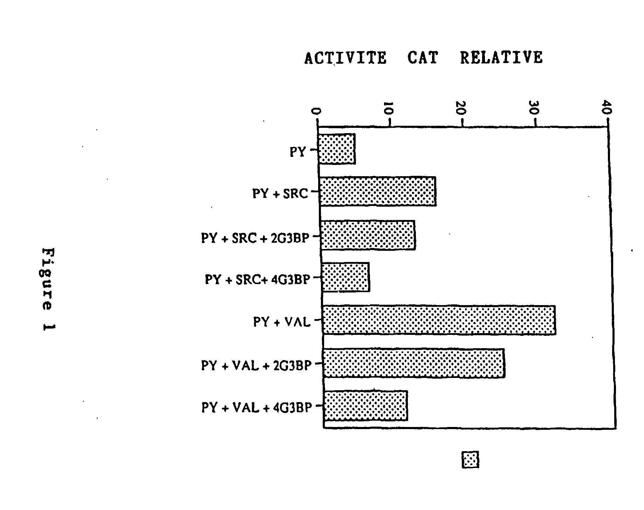

Au sens de la présente invention, le terme dérivé désigne toute molécule obtenue par modification de nature génétique et/ou chimique du polypeptide selon l'invention et conservant l'activité recherchée. Par modification de nature génétique et/ou chimique, on doit entendre toute mutation, substitution, délétion, addition et/ou modification d'un ou plusieurs résidus. De tels dérivés peuvent être générés dans des buts différents, tels que notamment celui d'augmenter l'affinité du peptide pour son site d'interaction, celui d'améliorer ses niveaux de production, celui d'augmenter sa résistance à des protéases, celui d'augmenter son efficacité thérapeutique ou de réduire ses effets secondaires, ou celui de lui conférer de nouvelles propriétés pharmacocinétiques et/ou biologiques.
Il peut également s'agir de fragments des séquences précitées de dérivés de celles-ci. De tels fragments peuvent être générés de différentes façons. En particulier, ils peuvent être synthétisés par voie chimique, sur la base de la séquence donnée dans la figure 1, en utilisant les synthétiseurs peptidiques connus de l'homme du métier. Ils peuvent également être synthétisés par voie génétique, par expression dans un hôte cellulaire d'une séquence nucléotidique codant pour le peptide recherché. Dans ce cas, la séquence nucléotidique peut être préparée chimiquement en utilisant un synthétiseur d'oligonucléotides, sur la base de la séquence peptidique donnée dans la présente demande et du code génétique. La séquence nucléotidique peut également être préparée à partir de la séquence donnée dans la présente demande, par coupures enzymatiques, ligature, clonage, etc, selon les techniques connues de l'homme du métier, ou par criblage de banques d'ADN avec des sondes élaborées à partir de la SEQ ID N°1.

Selon un mode particulier de l'invention, le polypetide revendiqué comprend la SEQ ID N°1, SEQ ID N°2, SEQ ID N°3 et/ou SEQ ID N°4.

De préférence, le polypeptide selon l'invention possède un poids moléculaire de l'ordre de 68kDa.

Selon un mode particulier de l'invention, il s'agit d'un polypeptide d'origine humaine comprenant tout ou partie de la SEQ ID N°5, de la SEQ ID N°9.

Il s'agit plus particulièrement d'un polypeptide comprenant la SEQ ID N°5.

Plus préférentiellement, il s'agit d'un polypeptide représenté en SEQ ID N°9.

De manière inattendue, cette protéine ne possède pas d'homologie avec une autre protéine p68 déjà identifiée comme se liant au domaine SH3 de Src. Elle n'est pas phosphorylée au niveau de ses motifs tyrosine dans les cellules en croissance ou à l'état de mitose.

L'analyse de la séquence protéique ID N°9 révèle que la protéine associée au domaine SR+H3 de la protéine GAP, désignée G3BP, appartient à la famille des hnRNP (heterogenous nuclear RiboNucleoProteins). Plus précisément G3BP est une protéine de 466 A.a, présentant un poids moléculaire apparent de 68 kDa qui contient plusieurs domaines caractéristiques des protéines qui se lient aux ARN :
- des domaines RNP2 et RNP1 (A.a 342 à 347)
- 4 RGG box (A.a 435 à 449)
- un domaine auxiliaire acide (A.a 144 à 221).

L'invention s'étend également aux peptides capables d'antagoniser l'interaction entre la G3BP et le domaine SH3 de GAP. L'activité de ces peptides peut être mise en évidence dans des tests de compétition (cf exemple 2-3) ou d'interférence des signaux transduits par les protéines Ras.

Un autre objet de l'invention réside dans des anticorps ou fragments d'anticorps polyclonaux ou monoclonaux dirigés contre un polypeptide tel que défini ci-avant. De tels anticorps peuvent être générés par des méthodes connues de l'homme du métier. En particulier, ces anticorps peuvent être préparés par immunisation d'un animal contre un polypeptide dont la séquence est choisie parmi les SEQ ID N°1, SEQ ID N°2, SEQ ID N°3, SEQ ID N°4, SEQ ID N°5 et la SEQ ID N°9 ou tout fragment ou dérivé de celles-ci, prélèvement du sang et isolement des anticorps. Ces anticorps peuvent également être générés par préparation d'hybridomes selon les techniques connues de l'homme de l'art.
Les anticorps ou fragments d'anticorps de l'invention peuvent ainsi être utilisés pour réguler l'état d'activation du produit des gènes ras.
Par ailleurs, ces anticorps peuvent également être utilisés pour détecter et/ou doser un peptide selon l'invention dans des échantillons biologiques, et de ce fait, pour renseigner sur l'état d'activation du produit des gènes ras.
L'invention s'étend également aux antagonistes à savoir tout peptide capable de bloquer l'interaction d'un polypeptide selon l'invention avec le domaine SH3 de la protéine GAP. De tels peptides peuvent être mis en évidence dans des tests de compétition (cf exemple 2-3) ou d'inhibition de l'activité ras.
La présente invention permet donc de générer des polypeptides dérivés des séquences identifiées ci-dessus ainsi que des anticorps dirigés contre ces polypeptides
ou des protéines correspondantes, présentant des propriétés biologiques interessantes en vue d'une utilisation pharmaceutique.
L'invention fournit également des composés non peptidiques ou non exclusivement peptidiques utilisables pharmaceutiquement. Il est en effet possible, à partir des motifs protéiques actifs décrits dans la présente demande, de réaliser des molécules inhibitrices de la voie de signalisation dépendante des protéines P21 non exclusivement peptidiques et compatibles avec une utilisation pharmaceutique. A cet égard, l'invention concerne l'utilisation d'un polypeptide de l'invention tel que décrit ci-avant pour la préparation de molécules non-peptidiques, ou non exclusivement peptidiques, actives pharmacologiquement, par détermination des éléments structuraux de ce polypeptide qui sont importants pour son activité et reproduction de ces éléments par des structures non-peptidiques ou non exclusivement peptidiques. L'invention a aussi pour objet des compositions pharmaceutiques comprenant une ou plusieurs molécules ainsi préparées.

La présente invention a également pour objet toute séquence d'acide nucléique codant pour un polypeptide capable de se lier au domaine SH3 de la protéine GAP. Plus préférentiellement, il s'agit d'une séquence comprenant :
(a) tout ou partie de la SEQ ID N°6, SEQ ID N°10 ou d'un de leurs brins complémentaires,
(b) les séquences dérivées des séquences (a) en raison de la dégénérescence du code génétique.

De préférence, elle comprend la séquence SEQ ID N°6 et plus préférentiellement est représentée par LA SEQ ID N°10.

Les différentes séquences nucléotidiques de l'invention peuvent être d'origine artificielle ou non. Il peut s'agir de séquences génomiques, d'ADNc, d'ARN, de séquences hybrides ou de séquences synthétiques ou semi-synthétiques. Ces séquences peuvent être obtenues par exemple par criblage de banques d'ADN (banque d'ADNc, banque d'ADN génomique) au moyen de sondes élaborées sur la base de séquences présentées ci-avant. De telles banques peuvent être préparées à partir de cellules de différentes origines par des techniques classiques de biologie moléculaires connues de l'homme du métier. Les séquences nucléotidiques de l'invention peuvent également être préparées par synthèse chimique, notamment selon la méthode des phosphoramidites, ou encore par des méthodes mixtes incluant la modification chimique ou enzymatique de séquences obtenues par criblage de banques.

Ces séquences nucléotidiques selon l'invention sont utilisables dans le domaine pharmaceutique, soit pour la production des polypeptides de l'invention, soit pour la réalisation de séquences antisens utilisables dans le cadre d'une thérapie génique, soit encore pour la détection et le diagnostic, par des expériences d'hybridation, de l'activité de la protéine GAP dans des échantillons biologiques ou pour l'isolement de séquences homologues à partir d'autres sources cellulaires.

Pour la production des polypeptides de l'invention, les séquences nucléiques définies ci-dessus sont généralement placées sous le contrôle de signaux permettant leur expression dans un hôte cellulaire. Le choix de ces signaux (promoteurs, terminateurs, sequence "leader" de sécrétion, etc) peut varier en fonction de l'hôte cellulaire utilisé. Préférentiellement, ces séquences nucléotidiques de l'invention font partie d'un vecteur, qui peut être à réplication autonome ou intégratif. Plus particulièrement, des vecteurs à réplication autonome peuvent être préparés en utilisant des séquences à réplication autonome chez l'hôte choisi. S'agissant des vecteurs intégratifs, ceux-ci peuvent être préparés par exemple en utilisant des séquences homologues à certaines régions du génome de l'hôte, permettant, par recombinaison homologue, l'intégration du vecteur.

Les hôtes cellulaires utilisables pour la production des polypeptides de l'invention sont aussi bien des hôtes eucaryotes que procaryotes. Parmi les hôtes eucaryotes qui conviennent, on peut citer les cellules animales, les levures, ou les champignons. En particulier, s'agissant de levures, on peut citer les levures du genre *Saccharomyces, Kluyveromyces, Pichia, Schwanniomyces,* ou *Hansenula.* S'agissant de cellules animales, on peut citer les cellules COS, CHO, C127, etc. Parmi les champignons, on peut citer plus particulièrement *Aspergillus* ssp. ou *Trichoderma* ssp. Comme hôtes procaryotes, on préfère utiliser les bactéries suivantes *E.coli, Bacillus,* ou *Streptomyces.*

Les séquences d'acides nucléiques selon l'invention peuvent également servir à la réalisation d'acides nucléiques antisens utilisables comme agents pharmaceutiques. L'inhibition de l'expression de certains oncogènes par des acides nucléiques antisens s'est avérée être une stratégie utile dans la compréhension du rôle de ces oncogènes et une voie particulièrement prometteuse dans la réalisation d'un traitement anticancéreux. Les oligonucléotides antisens sont des oligonucléotides de petite taille, complémentaires du brin codant d'un gène donné, et de ce fait capables d'hybrider spécifiquement avec l'ARNm transcrit, inhibant sa traduction en protéine. De tels oligonucléotides peuvent être constitués par tout ou partie des séquences nucléiques définies ci-avant. Il s'agit généralement de séquences ou de fragments de séquences complémentaires de séquences codant pour des peptides selon l'invention. De tels oligonucléotides peuvent être obtenus à partir des séquences précitées, par fragmentation, etc, ou par synthèse chimique.

L'invention concerne également, comme séquences nucléotidiques, les sondes nucléotidiques, synthétiques ou non, capables de s'hydrider avec les séquences nucléotidiques définies ci-avant qui codent pour un polypeptide de l'invention, ou avec l'ARNm correspondant. De telles sondes peuvent être utilisées in vitro comme outil de diagnostic. De telles sondes doivent être préalablement marquées, et pour cela différentes techniques sont connues de l'homme du métier. Les conditions d'hybridation dans lesquelles ces sondes peuvent être utilisées sont les conditions normales de stringence. Ces sondes peuvent également être utilisées pour la mise en évidence et l'isolement de séquences d'acides nucléiques homologues codant pour un polypeptide de l'invention, à partir d'autres sources cellulaires. A titre illustratif de ces sondes, on peut plus particulièrement mentionner les sondes représentées par les SEQ ID N°7 et SEQ ID N°8, qui sont utilisées en exemple 3 ci-après.

L'invention a encore pour objet toute composition pharmaceutique comprenant comme principe actif au moins un polypeptide tel que défini ci-avant
Elle a aussi pour objet toute composition pharmaceutique comprenant comme principe actif au moins un anticorps et/ou un fragment d'anticorps tel que défini ci-avant, ainsi que toute composition pharmaceutique comprenant comme principe actif au moins un oligonucléotide antisens tel que défini ci-avant.
Par ailleurs, elle a aussi pour objet les compositions pharmaceutiques dans lesquelles les polypeptides, anticorps et oligonucléotides antisens définis ci-avant sont associés entre-eux ou avec d'autres principes actifs.
Les compositions pharmaceutiques selon l'invention peuvent être utilisées pour moduler l'activation des protéines p21 et de ce fait pour moduler la prolifération de certains types cellulaires. Plus particulièrement, ces compositions pharmaceutiques sont destinées au traitement de cancers. De nombreux cancers ont en effet été associés à la présence de protéines ras oncogéniques. Parmi les cancers renfermant le plus souvent des gènes ras mutés, on peut citer notamment les adénocarcinomes du pancréas, dont 90% ont un oncogène Ki-ras muté sur le douzième codon (Almoguera et coll., Cell 53 (1988) 549), les adénocarcinomes du colon et les cancers de la thyroïde (50%), ou les carcinomes du poumon et les leucémies myéloïdes (30%, Bos, J.L. Cancer Res. 49 (1989) 4682).

L'invention a encore pour objet l'utilisation des molécules décrites ci-avant pour moduler voire inhiber l'activité des protéines p21. En particulier, l'invention a pour objet l'utilisation de tout ou fragment de G3BP pour interférer avec les signaux transduits par les produits des gènes Ras. Les fragments protéiques homologues aux hnRNP sont utilisés avantageusement pour inhiber la liaison de G3BP avec ses ARN cibles. Des séquences identiques ou complémentaires à ces ARN cibles peuvent être également utilisées pour interférer avec les signaux transduits par la protéine G3BP.

L'invention fournit également un procédé de détection de l'expression et/ou d'une surexpression de la protéine G3BP dans un échantillon biologique. Un tel procédé comprend par exemple la mise en contact d'un tel échantillon avec un anticorps ou fragment d'anticorps selon l'invention, la révélation des complexes antigène-anticorps, et la comparaison des résultats obtenus avec un échantillon standard. Dans un tel procédé, l'anticorps peut être en suspension ou préalablement immobilisé sur un support. Ce procédé peut également comprendre la mise en contact de l'échantillon avec une sonde nucléotidique selon l'invention, la mise en évidence des hybrides obtenus, et la comparaison avec ceux obtenus dans le cas d'un échantillon standard.

La présente invention peut être utilisée à plusieurs titres dans le domaine thérapeutique : les polypeptides, anticorps et séquences nucléotidiques de l'invention étant capables de moduler l'activité des gènes ras, ils permettent en effet d'intervenir dans le processus de développement des cancers. En raison de leur forte expression dans les cellules de muscles striés squelettique, ces peptides interviennent en outre vraisemblablement dans les pathologies liées à un défaut de signalisation comme le diabète par exemple. Un autre aspect de l'invention consiste à utiliser des séquences nucléotidiques ADN ou ARN capables d'interagir avec les polypeptides revendiqués. Ces séquences peuvent être préparées selon la méthode décrite dans la demande internationale WO 91/19813.

L'invention peut également être utilisée dans le domaine du diagnostic et du typage de cancers.

D'autres avantages de la présente invention apparaîtront à la lecture des exemples et figures qui suivent, qui doivent être considérés comme illustratifs et non limitatifs.

### FIGURES:

Figure 1: Effet d'une surexpression de G3BP dans des fibroblastes NIH 3T3 sur l'activité CAT.
Figure 2: Effet d'une surexpression de G3BP dans des fibroblastes NIH 3T3 sura formation de foyers induite par les oncogènes Src et Ras .

### MATERIEL ET METHODES

### Techniques générales de clonage

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extractions de protéines au phénol ou au phénol-chloroforme, la précipitation d'ADN en milieu salin par de l'éthanol ou de l'isopropanol, la transformation dans *Escherichia coli,* etc, sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].
Les enzymes de restriction ont été fournies par New England Biolabs (Biolabs), Bethesda Research Laboratories (BRL) ou Amersham et sont utilisées selon les recommandations des fournisseurs.
Le plasmide pGEX 2T est d'origine commerciale.
L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] est effectuée en utilisant un "DNA thermal cycler" (Perkin Elmer Cetus) selon les spécifications du fabricant
La vérification des séquences nucléotidiques est effectuée par la méthode développée par Sanger et al. [Proc. Natl. Acad. Sci. USA, 74 (1977) 5463-5467] en utilisant le kit distribué par Amersham.
Pour les expériences d'hybridation, les conditions de stringence normales sont généralement les suivantes : hybridation : 3 x SCC en présence de 5 x Denhart's à 65°C; lavage : 0,5 x SSC à 65 °C.

### EXEMPLE 1

### Préparation des protéines de fusion Glutathion-S-transférase SH3

Les séquences d'ADN codant pour les domaines SH3 des protéines GAP (résidus 275 à 350) et c-Src (résidus 84 à 148) sont amplifiées par la technique de P.C.R. et clonées dans un vecteur d'expression pGEX2T entre les sites de restriction Bam HI et ECORI. Les bactéries ansi transformées sont cultivées, induites avec du IPTG (1-thio-β-D galactopyranoside) et lysées par sonication. Les protéines de fusion GST SH3 sont purifiées par chromatographie d'affinité sur billes d'agarose glutathion (Pharmacia LKB biotech) puis éluées avec 10mM de glutathion réduit

### EXEMPLE 2

### 1)Préparation des lysats cellulaires

Des cellules ER22 (fibroblastes de hamster surexprimant le récepteur EGF humain) ou des cellules NIH 3T3 exprimant pp60 C-Src(F-527) sont cultivées sur milieu DMEM (Dubelcco's Modified Eagle Medium) enrichi avec 10% de sérum de veau foetal contenant l'antibiotique G418 (200µg/ml) et 2mM/l de glutamine (5GBICO-BRL). à 37°C sous 5% en CO₂.
Dans chaque essais, les cellules ER22 sont cultivées dans des boites de 100mm jusqu'à confluence puis sans sérum pendant 18heures. De l'orthovanadate de sodium est ajouté à une concentration finale de 100µM et l'incubation poursuivie pendant 30 minutes. L'EGF est ensuite ajouté directement au milieu pour une concentration finale de 80nM en 10 minutes et à 37°C.

Pour certains essais, les cellules mitotiques sont récupérées par traitement avec 0,4µg de nocodazole (SIGMA) par ml pendant 18heures. Elles sont rapidement lavées avec un tampon salin à base de phosphate, refroidi dans la glace, puis solubilisées en 30 minutes à 4°C dans 1 ml de tampon de lyse, HNTG (50 mM d'Hepès, pH 7,5, 150 mM de NaCl, 1% de Triton x 100, 10% de Glycérol, 1 mM de MgCl₂ , 1 mM d'EGTA, en présence d'inhibiteurs de phosphatases (1 mM de Na₃VO₄, 10 mM de Na₄P₂O₇, 10 mM de NaF) et d'inhibiteurs de protéases (1 µg/ml de leupeptine, 1 µg/ml d'inhibiteur de trypsine, 1 µg/ml de pepstatine A, 2 µg/ml d'aprotinine, 10 µg/ml de benzamidine, 1 mM de phénylméthanesulfonyle fluorure, 1 µg/mlµg/ml d'antipaïne, 1 µg/ml de chymostatine).

Les lysats sont décantés par centrifugation à 15 000 tours/mn pendant 10 mn. La concentration en protéines est ensuite déterminée (Bio-rad micro-test).

### 2) Test de liaisons directes

L'ensemble des lysats cellulaires (200 µg) et des protéines immunoprécipitées sont séparés sur gel de polyacrylamide à 7,5% de dodécyl sulfate de sodium (SDS-PAGE) puis transférés sur une membrane de difluorure de polyvinylidène (PVDF Millipore Corpo.). La liaison non spécifique au niveau des filtres est bloquée par 2% de lait écrémé dans du PBS contenant 0,05% de Tween 20, pendant 2 heures, à température ambiante. Les filtres sont incubés avec la protéine GST et des protéines GST-SH3 dans le tampon bloquant pendant 12H à 4°C. Après avoir été lavées avec du PBS-0,05% Tween 20, les protéines liées sont détectées par incubation successive avec un anticorps monoclonal anti-GST (0,25 µg/ml) (Hybridolab Pasteur Inst.), un anticorps anti-souris conjugué à une phosphatase alcaline et du sel 5 bromo-4 chloro-3 indolyphosphate toluidinium nitroblue tetrazolium (PROMEGA).

### 3)Test de compétition

Des peptides synthétiques correspondant aux séquences (275-305), (299-326), (317-326), (320-350) de GAP SH3 ou à la séquence putative de la dynamine (RRAPAVPPARPGS) se liant au domaine SH3 sont synthétisés sur un appareil Applied Biosystems 431 A, utilisant la chimie FMOC.
La protéine p68 purifiée est isolée par électrophorèse sur gel de polyacrylamide-dodécyl sulfate de sodium et transférée par électrophorèse sur membrane PVDF. Les membranes sont incubées avec des quantités croissantes en peptides. Le peptide Poly-L-proline (SIGMA) est utilisé à 500 µM dans une réaction témoin. Après 1 H d'incubation, la protéine GST-GAP-SH3 (2µg/ml) est ajouté à chaque milieu réactionnel. Les filtrats sont sondés avec un anticorps monoclonal anti-GST comme décrit précédemment

### 4)Immunoprécipitation et immunotransfert

Les lysats cellulaires solubles (3 mg) sont décantés avec 50 µl de protéine A sépharose CL-4B (Pharmacia Biotech) pendant 2 H à 4 °C. Les lysats cellulaires décantés sont incubés avec l'anticorps monoclonal antiphosphotyrosine (anticorps monoclonal 4G10 - Upstate Biotechnology Incorporated) pendant 4 H à 4 °C. Puis, 50 µl de protéine A-Sépharose sont ajoutés au complexe et l'incubation est poursuivie toute une nuit à 4°C. Les immunoprécipités sont lavés 3 fois avec un tampon HNTG et solubilisés dans un échantillon de tampon SDS (100 µl). Les complexes sont ensuite séparés par SDS-PAGE et transférés par électrophorèse sur des membranes PVDF.

Les membranes sont incubées avec l'anticorps monoclonal phosphotyrosine dans du TBS (10 mM de Tris, pH 7,4.150 mM de NaCl, 3% de sérum albumine bovin) et en outre incubées avec des seconds anticorps conjugués à une phosphatase alcaline. Des substrats pour la phosphatase alcaline sont ensuite ajoutés pour un développement approprié de la couleur.

### 5)Purification et analyse de la séquence

Environ 5.10⁹ cellules ER22 sont lysées dans 200 ml de tampon HNTG. Le lysat est centrifugé à 15 000 g pendant 15 min et dilué 5 fois dans un tampon HNG (50 mM d'Hepes, pH 7,5, 150 mM de NaCl, 10% de Glycérol, 1 mM d'EGTA, inhibiteurs de phosphatase et inhibiteurs de protéase). Le lysate est incubé toute une nuit avec 6 ml de S-Sépharose Fast flow équilibré dans le même tampon. Le complexe est transvasé sur colonne (2,5 x 1,3 cm - IBF). La colonne est lavée avec 10 fois son volume en tampon et les protéines liées sont ensuite éluées à une vitesse d'élution de 60 ml h-1, avec un gradient linéaire de 60 ml de 0 à 1 M de NaCl du même tampon. Les fractions possédant l'activité de liaison déterminée dans les conditions de l'exemple 2.2 (0,15-0,37 M NaCl) sont rassemblées, diluées 10 fois dans un tampon HNG et chargées sur Héparine-Sépharose CL-6B (3ml) (Pharmacia LKB), prééquilibrée avec le même tampon à une vitesse d'élution de 24 ml h-1. Après lavage avec le tampon HNG, la colonne est éluée avec un gradient de 24 ml, de 0 à 1 M de NaCl. Des fractions actives de la chromatographie S Fast Flow sont rassemblées, diluées 4 fois dans un tampon MES (100 mM de MES, ph 6,8, 1 mM de MgSO₄. 1 mM d'EGTA) et transférées sur une colonne ATP d'agarose (3 ml) (Sigma N° A9264) prééquilibrée avec le tampon MES contenant 50 mM de NaCl à un débit d'élution de 6 ml.h-1. La colonne est ensuite lavée avec 20 ml de tampon MES contenant 50 mM de NaCl et éluée à 30 mLh-1 avec un gradient linéaire de 50 mM à 2 M NaCl dans le tampon MES. La protéine p68 élue entre 0.3 M et 0.4 M de NaCl. Les fractions actives sont rassemblées, dialysées avec 20 mM de NH₄HCO₃ pH 8,3 et concentrées. Les protéines amenées à sec, sont resuspendues dans un tampon SDS, séparées par électrophorèse sur gel de polyacrylamide. Le gel est coloré avec du bleu de coomassie et la bande de poids moléculaire de 68kDA, correspondant à l'activité de liaison SH₃ est recueillie. Elle est lavée pendant 1 heure avec les solutions suivantes: eau, eau/méthanol (90/10), eau/CH3CN (80/20), eau/CH3CN (50/50).

La bande de gel, contenant la protéine p68 purifiée, est ensuite divisée en petits fragments et séchée sous SPEED/VAC (SAVANT). On ajoute 400µl d'une solution contenant 25 mM Tris pH 8,5, 1 mM d'EDTA , 0,05% de SDS et 5µg d'endoprotéinase Lys-c (Boehringer Mannheim) et l'ensemble est incubé toute une nuit à 37°C. L'hydrolysat est injecté sur colonne HPLC en phase inverse (Vydac C18: 2,1 x 250mm). La colonne est éluée à 0,2ml/min avec un gradient linéaire de 0 à 35% B en 150 minutes. (A:H2O + TFA 0,07% et B: CH3CN + TFA 0,07%) et les pics d'élution observés à tr 113,7, 117,7 et 133,7 min sont recueillis et directement séquences en utilisant un microséquenceur Applied Biosystems 477A. La séquence des peptides correspondants ainsi obtenus est présentée en SEQ ID N°1, SEQ ID N°2, SEQ ID N°3 et SEQ ID N°4. Ces séquences ne présentent aucune homologie avec des protéines référencées dans les bases de données de protéines (PIR 33. Swiss-Prot 33 (intelligenetics)).

Afin de déterminer si la protéine p68 est phosphorylée au niveau de la tyrosine dans les cellules mitotiques ou non synchrones, les protéines des lysats issus de cellules ER22 cultivées dans 10% de sérum de veau foétal ou de cellules traitées avec le nocodazole sont immunoprécipitées avec des anticorps anti-phosphotyrosine, transférées sur membrane et soit immunodétectées avec des anticorps anti-phosphotyrosine soit incubées avec soit GST-GAP-SH3 ou GST-Src-SH3, dans les conditions décrites en exemples 2.2 et 2.4.
Comme témoin, les protéines des cellules NIH 3T3, transformées avec un allèle activé de c-Src (c-Src Y527F) sont testées dans les mêmes conditions que celles décrites pour les protéines ER22. Les résultats obtenus montrent que des protéines sont phosphorylées au niveau de la tyrosine, plus particulièrement dans la région 60-70kDa. Dans les cellules ER22 aucune liaison à une protéine phosphorylée p68 n'est détectée avec les sondes GST-GAP-SH3 ou GST-Src-SH3. Dans les cellules NIH3T3 (c-Src Y527F), la sonde GST-Src-SH3 se lie à une protéine de poids moléculaire de 68kDa phosphorylée en tyrosine alors que la sonde GST-GAP-SH3 n'interéagit avec aucune protéine.

Afin de confirmer l'implication fonctionnelle de cette protéine dans la voie de signalisation ras, des expériences de compétition ont été réalisées dans les conditions décrites dans l'exemple 2.3. Les résultats obtenus montrent que les peptides dérivés de GAP capables de bloquer la rupture des vésicules germinales d'oeufs de xénope (GVBD) induite par ras, peptides (299-326) et (317-326) de GAPSH3 sont également capables de bloquer l'interaction entre la protéine G3BP et GAP.
Ces résultats confirment clairement que la capacité de bloquer l'activité de la protéine selon l'invention ou d'interférer avec son activité constitue une nouvelle approche particulièrement prometteuse pour le traitement du cancer.

### EXEMPLE 3

### Isolement de la séquence SEQ ID N°6 humaine à partir d'une banque d'ADNc de placenta humain.

On utilise à titre de sondes les SEQ ID N°7 et N°8, établies à partir des séquences peptidiques SEQ ID N°1 et SEQ ID N°3. L'ADN de la banque Clonetech HL1008B a été préparé et utilisé dans une réaction de PCR. 30 cycles d'amplification ont été réalisés dans les conditions suivantes: dénaturation 1 min à 94°C, appariement 1 min entre 35 et 40°C et élongation 1 min à 72°C. Cette réaction permet d'isoler un fragment d'ADN de 1,3kb dont une partie de la séquence est donnée en SEQ ID N°6. Une analyse en Northern Blot montre que l'ARN correspondant (3,3kb) est ubiquitaire et exprimé très fortement dans le muscle squelettique adulte.

### EXEMPLE 4 :

### Isolement de la séquence SEQ ID N°10 codant pour la p68 humaine à partir d'une banque d'ADNc de placenta humain.

On utilise les deux oligonucléotides (SEQ ID N°7) et (SEQ ID N°8) comme amorces pour l'amplication d'un ADNc dans une banque de placenta humain. La P.C.R. a été réalisée grâce à l'Amplitaq Perkin Elmer à une température d'annealing de 35°C suivie d'1mn d'extension à 72°C en présence de 10 % de formamide. Nous avons amplifié un fragment d'ADNc de 1164pb. Après un clonage direct en vecteur pMOSblue (Amersham) comportant des séquences -20 et inverse, le fragment a été séquencé grâce à des sondes fluorescentes. Ce fragment PCR a ensuite été utilisé comme sonde pour screener 10⁶ phages d'une banque λgt11 d'ADNc de placenta humain (Clontech). La sonde a été synthétisée par le système Rediprime d'Amersham et les filtres ont été incubés 16 heures dans le tampon d'hybridation (6X SSC, 5X Denhardt's, 100 µg/ml sperme de saumon, 0,25 % SDS) contenant la sonde à 45°C. Les filtres ont ensuite été lavés 1 heure à température ambiante et 20 mn à température d'hybridation en 2X SSC, 0;05 % SDS. Huit clones positifs ont été identifiés. Deux d'entres eux ont été purifiés et les ADNc ont été digéré par EcoRI pour ressortir les inserts. Ils ont été sous-clonés en M13mp18/EcoRI pour être séquencés. Le séquençage a été réalisé sur les fragments issus d'une délétion progressive à l'exonucléase III de l'ADNc (Nested Deletion Kit, Pharmacia Biotech). Les différences de taille des fragments ont été analysées par amplification PCR entre les amorces -20 et reverse du vecteur M13 et différentes populations de taille ont été choisies pour subir le séquençage. L'assemblage des différentes séquences obtenues a permis la reconstitution de la phase ouverte entière de la P68.

### EXEMPLE 5

### Surexpression de la G3BP dans des fibroblastes NIH 3T3

Des fibroblastes NIH 3T3 sont transfectés par un gène rapporteur, celui de la chloramphénicol acétyl transférase, placé sous contrôle d'éléments de réponse à Ras dérivés de l'enhancer du virus du polyôme. Ces éléments sont stimulés de 15 à 30 fois lorsque les cellules sont transfectées par un vecteur d'expression portant l'ADNc des oncogènes Src et Ras. Ces stimulations sont affectées lorsque la protéine G3BP est exprimée après cotransfection d'un vecteur d'expression contenant un ADNc qui correspond à la phase ouverte de la protéine. De façon dose-dépendant, la G3BP inhibe l'activité CAT stimulée par Src et par la forme oncogénique de Ras. Cette observation est représentée en figure 1.

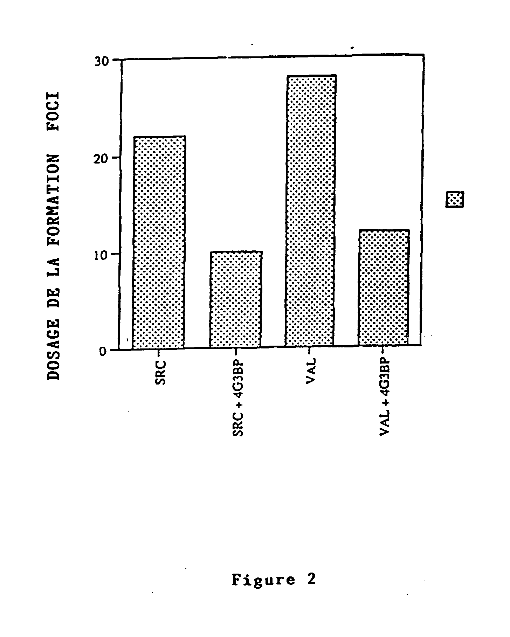

De la même façon, l'expression de la protéine G3BP s'oppose à la formation de foyers induite par les oncogènes Src et Ras. La figure 2 rend compte de cette observation.

Ces expériences démontrent clairement la capacité de G3BP à s'opposer aux effets prolifératifs des signaux transduits par les protéines Ras normales ou oncogéniques.

## Revendications

1. Polypeptide capable d'interagir avec le domaine SH3 de la protéine GAP **caractérisé en ce qu'**il comprend une séquence peptidique choisie parmi les séquences SEQ ID N°1, SEQ ID N°2, SEQ ID N°3, SEQ N°4, SEQ ID N°5, ou la SEQ ID N°9.

2. Polypeptide selon la revendication 1, **caractérisé en ce qu'**il comprend la SEQ ID N°1, SEQ ID N°2, SEQ ID N°3 et/ou SEQ ID N°4.

3. Polypeptide selon l'une des revendications précédentes **caractérisé en ce qu'**il possède un poids moléculaire de l'ordre de 68kDa.

4. Polypeptide selon la revendication 1 ou 3 **caractérisé en ce qu'**il est d'origine humaine.

5. Polypeptide selon la revendication 4 **caractérisé en ce qu'**il comprend la séquence SEQ ID N°5 ou la SEQ ID N°9.

6. Polypeptide selon la revendication 1, 4 ou 5 **caractérisé en ce qu'**il est représenté par la SEQ ID N°9.

7. Polypeptide selon l'une des revendications précédentes **caractérisé en ce que** ses motifs tyrosine ne se phosphorylent pas dans des cellules mitotiques ou en croissance.

8. Anticorps ou fragment d'anticorps **caractérisé en ce qu'**il est dirigé contre une séquence choisie parmi les séquences peptidiques présentées en SEQ ID N°1, SEQ ID N°2, SEQ ID N°3 , SEQ ID N°4, SEQ ID N°5, SEQ ID N°9.

9. Anticorps ou fragment d'anticorps selon la revendication 8 **caractérisé en ce qu'**il possède la faculté d'inhiber l'interaction entre la protéine GAP et un polypeptide selon l'une des revendications 1 à 7.

10. acide nucleique **caractérisé en ce qu'**il comprend :
(a) la séquence SEQ ID N°6 ou la SEQ ID N°10 ou leurs brins complémentaires, et
(b) les séquences dérivées des séquences (a) en raison de la dégénérescence du code génétique.

11. Séquence nucléotidique selon la revendication 10 **caractérisée en ce qu'**elle comprend la séquence SEQ ID N°6.

12. Acide nucléique selon la revendication 10 ou 11 **caractérisé en ce qu'**il est représenté en SEQ ID N°10

13. Utilisation d'un acide nucléique selon l'une des revendications 10 à 12 pour le diagnostic d'anomalies génétiques.

14. Composition pharmaceutique comprenant comme principe actif au moins un polypeptide selon l'une des revendications 1 à 7 et/ou un anticorps ou fragment d'anticorps selon la revendication 8 ou 9.

15. Composition pharmaceutique selon la revendication 14 destinée à moduler l'activation des protéines p21.

16. Composition pharmaceutique selon la revendication 14 destinée au traitement des cancers.

17. Utilisation d'un anticorps ou d'un fragment d'anticorps selon la revendication 8 pour la détection de l'expression et/ou d'une surexpression d'un polypeptide selon l'une des revendications 1 à 7.

18. Utilisation d'un polypeptide selon l'une des revendications 1 à 7 pour la préparation d'un médicament destiné à interférer avec les signaux transduits par les produits des gènes Ras.

## Patentansprüche

1. Polypeptid, das fähig ist, mit der Domäne SH3 des GAP-Proteins zu interagieren, **dadurch gekennzeichnet, dass** es eine Peptidsequenz umfasst, die aus den Sequenzen SEQ ID NO:1, SEQ ID NO:2, SEQ ID NO:3, SEQ ID NO:4, SEQ ID NO:5 oder SEQ ID NO:9 ausgewählt ist.

2. Polypeptid nach Anspruch 1, **dadurch gekennzeichnet, dass** es die SEQ ID NO:1, SEQ ID NO:2, SEQ ID NO:3 und/oder SEQ ID NO:4 umfasst.

3. Polypeptid nach einem der vorangehenden Ansprüche, **dadurch gekennzeichnet, dass** es ein Molekulargewicht in der Größenordnung von 68 kDa besitzt.

4. Polypeptid nach Anspruch 1 oder 2, **dadurch gekennzeichnet, dass** es humanen Ursprungs ist

5. Polypeptid nach Anspruch 4, **dadurch gekennzeichnet, dass** es die Sequenz SEQ ID NO:5 oder SEQ ID NO:9 umfasst.

6. Polypeptid nach Anspruch 1, 4 oder 5, **dadurch gekennzeichnet, dass** es durch SEQ ID NO:9 dargestellt wird.

7. Polypeptid nach einem der vorangehenden Ansprüche, **dadurch gekennzeichnet, dass** seine Tyrosinmotive in mitotischen Zellen oder Zellen im Wachstum nicht phosphoryliert werden.

8. Antikörper oder Antikörperfragment, **dadurch gekennzeichnet, dass** er/es gegen eine Sequenz gerichtet ist, die unter den Peptidsequenzen ausgewählt ist, welche in SEQ ID NO:1, SEQ ID NO:2, SEQ ID NO:3, SEQ ID NO:4, SEQ ID NO:5, SEQ ID NO:9 gezeigt sind.

9. Antikörper oder Antikörperfragment nach Anspruch 8, **dadurch gekennzeichnet, dass** er/es die Fähigkeit besitzt, die Wechselwirkung zwischen dem GAP-Protein und einem Polypeptid gemäß einem der Ansprüche 1 bis 7 zu inhibieren.

10. Nukleinsäure, **dadurch gekennzeichnet, dass** sie umfasst:
(a) die Sequenz SEQ ID NO:6 oder SEQ ID NO:10 oder ihre komplementären Stränge und
(b) die Sequenzen, die von den Sequenzen (a) infolge der Degeneriertheit der genetischen Codes abgeleitet sind.

11. Nukleotidsequenz nach Anspruch 10, **dadurch gekennzeichnet, dass** sie die Sequenz SEQ ID NO:6 umfasst.

12. Nukleinsäure nach Anspruch 10 oder 11, **dadurch gekennzeichnet, dass** sie in SEQ ID NO:10 dargestellt ist.

13. Verwendung einer Nukleinsäure nach einem der Ansprüche 10 bis 12 für die Diagnose von genetischen Anomalien.

14. Pharmazeutische Zusammensetzung, die als Wirkstoff wenigstens ein Polypeptid nach einem der Ansprüche 1 bis 7 und/oder einen Antikörper oder ein Antikörperfragment nach Anspruch 8 oder 9 umfasst.

15. Pharmazeutische Zusammensetzung nach Anspruch 14, die dazu bestimmt ist, die Aktivierung der Proteine p21 zu modulieren.

16. Pharmazeutische Zusammensetzung nach Anspruch 14, die zur Behandlung von Krebs bestimmt ist.

17. Verwendung eines Antikörpers oder eines Antikörperfragments nach Anspruch 8 zur Detektion der Expression und/oder einer Überexpression eines Polypeptids nach einem der Ansprüche 1 bis 7.

18. Verwendung eines Polypeptids nach einem der Ansprüche 1 bis 7 zur Herstellung eines Medikaments, das dazu bestimmt ist, mit den durch die Produkte der Ras-Gene translatierten Signalen zu interferieren.

## Claims

1. Polypeptide capable of interacting with the SH3 domain of the GAP protein, **characterized in that** it comprises a peptide sequence selected from among the sequences SEQ ID No. 1, SEQ ID No. 2, SEQ ID No. 3, SEQ No. 4, SEQ ID No. 5 or SEQ ID No. 9.

2. Polypeptide according to Claim 1, **characterized in that** it comprises SEQ ID No. 1, SEQ ID No. 2, SEQ ID No. 3 and/or SEQ ID No. 4.

3. Polypeptide according to either of the preceding claims, **characterized in that** it has a molecular weight of the order of 68 kDa.

4. Polypeptide according to Claim 1 or 3, **characterized in that** it is of human origin.

5. Polypeptide according to Claim 4, **characterized in that** it comprises the sequence SEQ ID No. 5 or SEQ ID No. 9.

6. Polypeptide according to Claim 1, 4 or 5, **characterized in that** it is represented by SEQ ID No. 9.

7. Polypeptide according to one of the preceding claims, **characterized in that** its tyrosine motifs are not phosphorylated in mitotic or growing cells.

8. Antibody or antibody fragment **characterized in that** it is directed against a sequence selected from among the peptide sequences which are depicted in SEQ ID No. 1, SEQ ID No. 2, SEQ ID No. 3, SEQ ID No. 4, SEQ ID No. 5 and SEQ ID No. 9.

9. Antibody or antibody fragment according to Claim 8, **characterized in that** it has the ability to inhibit the interaction between the GAP protein and a polypeptide according to one of Claims 1 to 7.

10. Nucleic acid **characterized in that** it comprises:
(a) the sequence SEQ ID No. 6 or SEQ ID No. 10 or their complementary strands, and
(b) the sequences which are derived from sequences (a) on account of the degeneracy of the genetic code.

11. Nucleotide sequence according to Claim 10, **characterized in that** it comprises the sequence SEQ ID No. 6.

12. Nucleic acid according to Claim 10 or 11, **characterized in that** it is depicted in SEQ ID No. 10.

13. Use of a nucleic acid according to one of Claims 10 to 12 for the diagnosis of gene anomalies.

14. Pharmaceutical composition which comprises, as the active principle, at least one polypeptide according to one of Claims 1 to 7 and/or an antibody or antibody fragment according to claim 8 or 9.

15. Pharmaceutical composition according to Claim 14 which is intended to modulate activation of the p21 proteins.

16. Pharmaceutical composition according to Claim 14 which is intended for treating cancers.

17. Use of an antibody or antibody fragment according to Claim 8 for detecting the expression and/or overexpression of a polypeptide according to one of Claims 1 to 7.

18. Use of a polypeptide according to one of Claims 1 to 7 for preparing a medicament intended to interfere with the signals which are transduced by the products of the Ras genes.


### LISTE DE SEQUENCES

(1) INFORMATION GENERALE:
   (i) DEPOSANT:
      (A) NOM: RHONE-POULENC RORER S.A.
      (B) RUE: 20, avenue Raymond ARON
      (C) VILLE: ANTONY
      (E) PAYS: FRANCE
      (F) CODE POSTAL: 92165
   (ii) TITRE DE L'INVENTION: PEPTIDE CAPABLE DE SE LIER AU DOMAINE SH3 DE LA PROTEINE GAP.
   (iii) NOMBRE DE SEQUENCES:10
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 19 acides aminés
      (B) TYPE: acide aminé
      (D) CONFIGURATION: linéaire
   (ii) TYPE DE MOLECULE: Protéine
   (vi) ORIGINE: HAMSTER
   (xi) DESCRIPTION DE LA SEQUENCE: SEQ ID NO: 1:
   Val Met Glu Lys Pro Ser Pro Leu Leu Val Gly Arg Glu Phe Val Arg Gln
   1               5                   10                  15
   Tyr Ile
(2) INFORMATION POUR LA SEQ ID NO: 2:
   (i) CARACTERISTIQUES DE LA SEQUENCE:
      (A) LONGUEUR: 15 acides aminés
      (B) TYPE: acide aminé
      (D) CONFIGURATION: linéaire
   (ii) TYPE DE MOLECULE: Protéine
   (vi) ORIGINE: HAMSTER
   (ix) FEATURE:
      (D) OTHER INFORMATION: Le premier Xaa représente soit un motif alanine ou thréonine et le second Xaa un acide aminé quelconque.
   (xi) DESCRIPTION DE LA SEQUENCE: SEQ ID NO: 2:
(2) INFORMATION POUR LA SEQ ID NO: 3:
   (i) CARACTERISTIQUES DE LA SEQUENCE:
      (A) LONGUEUR: 17 acides aminés
      (B) TYPE: acide aminé
      (D) CONFIGURATION: linéaire
   (ii) TYPE DE MOLECULE: Protéine
   (vi) ORIGINE: HAMSTER
   (xi) DESCRIPTION DE LA SEQUENCE: SEQ ID NO: 3:
   Leu Pro Asn Phe Gly Phe Val Val Phe Asp Asp Ser Glu Pro Val
   1               5                   10                  15
   Gln Lys
(2) INFORMATION POUR LA SEQ ID NO: 4:
   (i) CARACTERISTIQUES DE LA SEQUENCE:
      (A) LONGUEUR: 19 acides aminés
      (B) TYPE: acide aminé
      (D) CONFIGURATION: linéaire
   (ii) TYPE DE MOLECULE: Protéine
   (vi) ORIGINE: HAMSTER
   (xi) DESCRIPTION DE LA SEQUENCE: SEQ ID NO: 4:
   Ser Ala Thr Pro Ala Pro Ala Asp Val Ala Pro Ala Gln Glu Asp Leu
   1               5                   10                  15
   Arg Xaa Phe
(2) INFORMATION POUR LA SEQ ID NO: 5:
   (i) CARACTERISTIQUES DE LA SEQUENCE:
      (A) LONGUEUR: 68 acides aminés
      (B) TYPE: acide aminé
      (D) CONFIGURATION: linéaire
   (ii) TYPE DE MOLECULE: Protéine
   (vi) ORIGINE: HUMAIN
   (xi) DESCRIPTION DE LA SEQUENCE: SEQ ID NO: 5:
   Arg Glu Ala Gly Glu Gln Gly Asp Ile Glu Pro Arg Arg Met Val Arg
   1               5                   10                  15
   His Pro Asp Ser His Gln Leu Phe Ile Gly Asn Leu Pro His Glu Val
               20                  25                  30
   Asp Lys Ser Glu Leu Lys Asp Phe Phe Gln Ser Tyr Gly Asn Val Val
           35                  40                  45
   Glu Leu Arg Ile Asn Ser Gly Pro Lys Leu Pro Asn Phe Ala Phe Val
       50                  55                  60
   Val Phe Asp Asp
   65
(2) INFORMATION POUR LA SEQ ID NO: 6:
   (i) CARACTERISTIQUES DE LA SEQUENCE:SEQ ID NO: 6:
      (A) LONGUEUR:204 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Humain
   (xi) DESCRIPTION DE LA SEQUENCE: SEQ ID NO: 6:
   CGTGAGGCTG GTGAGCAAGG TGACATTGAA CCCCGAAGAA TGGTGAGACA CCCTGACAGT 60
   CACCAACTCT TCATTGGCAA CCTGCCTCAT GAAGTGGACA AATCAGAGCT TAAAGATTTC 120
   TTTCAAAGTT ATGGAAACGT GGTGGAGTTG CGCATTAACA GTGGTGGGAA ATTACCCAAT 180
   TTCGCCTTCG TCGTCTTCGA TGAT                                        204
(2) INFORMATION POUR LA SEQ ID NO: 7:
   (i) CARACTERISTIQUES DE LA SEQUENCE:SEQ ID NO: 7:
      (A) LONGUEUR:32 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Humain
   (xi) DESCRIPTION DE LA SEQUENCE: SEQ ID NO: 7:
   GTIATGGAIA AICCITCCCC ICTICTIGTI GG 32
(2) INFORMATION POUR LA SEQ ID NO: 8:
   (i) CARACTERISTIQUES DE LA SEQUENCE:SEQ ID NO: 8:
      (A) LONGUEUR:26 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Humain
   (xi) DESCRIPTION DE LA SEQUENCE: SEQ ID NO: 8:
   GAATCATCGA AIACIACGAA ICCGAA 26
(2) INFORMATIONS POUR LA SEQ ID NO: 9:
   (i) CARACTERISTIQUES DE LA SEQUENCE:
      (A) LONGUEUR: 466 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 9:
   Met Val Met Glu Lys Pro Ser Pro Leu Leu Val Gly Arg Glu Phe Val
   1               5                   10                  15
   Arg Gln Tyr Tyr Thr Leu Leu Asn Gln Ala Pro Asp Met Leu His Arg
               20                  25                  30
   Phe Tyr Gly Lys Asn Ser Ser Tyr Val His Gly Gly Leu Asp Ser Asn
           35                  40                  45
   Gly Lys Pro Ala Asp Ala Val Tyr Gly Gln Lys Glu Ile His Arg Lys
       50                  55                  60
   Val Met Ser Gln Asn Phe Thr Asn Cys His Thr Lys Ile Arg His Val
   65                  70                  75                  80
   Asp Ala His Ala Thr Leu Asn Asp Gly Val Val Val Gln Val Met Gly
                   85                  90                  95
   Leu Leu Ser Asn Asn Asn Gln Ala Leu Arg Arg Phe Met Gln Thr Phe
               100                 105                 110
   Val Leu Ala Pro Glu Gly Ser Val Ala Asn Lys Phe Tyr Val His Asn
           115                 120                 125
   Asp Ile Arg Tyr Gln Asp Glu Val Phe Gly Gly Phe Val Thr Glu Pro
   130 135 140
   Gln Glu Glu Ser Glu Glu Glu Val Glu Glu Pro Glu Glu Arg Gln Gln
   145 150 155 160
   Thr Pro Glu Val Val Pro Asp Asp Ser Gly Thr Phe Tyr Asp Gln Ala
   165 170 175
   Val Val Ser Asn Asp Met Glu Glu His Leu Glu Glu Pro Val Ala Glu
   180 185 190
   Pro Glu Pro Asp Pro Glu Pro Glu Pro Glu Gln Glu Pro Val Ser Glu
   195 200 205
   Ile Gln Glu Glu Lys Pro Glu Pro Val Leu Glu Glu Thr Ala Pro Glu
   210 215 220
   Asp Ala Gln Lys Ser Ser Ser Pro Ala Pro Ala Asp Ile Ala Gln Thr
   225 230 235 240
   Val Gln Glu Asp Leu Arg Thr Phe Ser Trp Ala Ser Val Thr Ser Lys
   245 250 255
   Asn Leu Pro Pro Ser Gly Ala Val Pro Val Thr Gly Ile Pro Pro His
   260 265 270
   Val Val Lys Val Pro Ala Ser Gln Pro Arg Pro Glu Ser Lys Pro Glu
   275 280 285
   Ser Gln Ile Pro Pro Gln Arg Pro Gln Arg Asp Gln Arg Val Arg Glu
   290 295 300
   Gln Arg Ile Asn Ile Pro Pro Gln Arg Gly Pro Arg Pro Ile Arg Glu
   305 310 315 320
   Ala Gly Glu Gln Gly Asp Ile Glu Pro Arg Arg Met Val Arg His Pro
   325 330 335
   Asp Ser His Gln Leu Phe Ile Gly Asn Leu Pro His Glu Val Asp Lys
   340 345 350
   Ser Glu Leu Lys Asp Phe Phe Gln Ser Tyr Gly Asn Val Val Glu Leu
   355 360 365
   Arg Ile Asn Ser Gly Gly Lys Leu Pro Asn Phe Gly Phe Val Val Phe
   370 375 380
   Asp Asp Ser Glu Pro Val Gln Lys Val Leu Ser Asn Arg Pro Ile Met
   385 390 395 400
   Phe Arg Gly Glu Val Arg Leu Asn Val Glu Glu Lys Lys Thr Arg Ala
   405 410 415
   Ala Arg Glu Gly Asp Arg Arg Asp Asn Arg Leu Arg Gly Pro Gly Gly
   420 425 430
   Pro Arg Gly Gly Leu Gly Gly Gly Met Arg Gly Pro Pro Arg Gly Gly
   435 440 445
   Met Val Gln Lys Pro Gly Phe Gly Val Gly Arg Gly Leu Ala Pro Arg
   450 455 460
   Gln Glx
   465
(2) INFORMATIONS POUR LA SEQ ID NO: 10:
   (i) CARACTERISTIQUES DE LA SEQUENCE:
      (A) LONGUEUR: 2129 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (xi) DESCRIPTION DE LA SEQUENCE: SEQ ID NO: 10:
   GCTTGCCTGT CAGGTCGACT CTAGAGCCCG GGTACCGAGC TCGAATTCGG CGGGGTTTGT 60
   ACTATCCTCG GTGCTGTGGT GCAGAGCTAG TTCCTCTCCA GCTCAGCCGC GTAGGTTTGG 120
   ACATATTTAC TCTTTTCCCC CCAGGTTGAA TTGACCAAAG CAATGGTGAT GGAGAAGCCT 180
   AGTCCCCTGC TGGTCGGGCG GGAATTTGTG AGACAGTATT ACACACTGCT GAACCAGGCC 240
   CCAGACATGC TGCATAGATT TTATGGAAAG AACTCTTCTT ATGTCCATGG GGGATTGGAT 300
   TCAAATGGAA AGCCAGCAGA TGCAGTCTAC GGACAGAAAG AAATCCACAG GAAAGTGATG 360
   TCACAAAACT TCACCAACTG CCACACCAAG ATTCGCCATG TTGATGCTCA TGCCACGCTA 420
   AATGATGGTG TGGTAGTCCA GGTGATGGGG CTTCTCTCTA ACAACAACCA GGCTTTGAGG 480
   AGATTCATGC AAACGTTTGT CCTTGCTCCT GAGGGGTCTG TTGCAAATAA ATTCTATGTT 540
   CACAATGATA TCTTCAGATA CCAAGATGAG GTCTTTGGTG GGTTTGTCAC TGAGCCTCAG 600
   GAGGAGTCTG AAGAAGAAGT AGAGGAACCT GAAGAAAGCA GCAAACACCT GAGGTGGTAC 660
   CTGATGATTC TGGAACTTTC TATGATCAGG CAGTTGTCAG TAATGACATG GAAGAACATT 720
   TAGAGGAGCC TGTTGCTGAA CCAGAGCCTG ATCCTGAACC AGAACCAGAA CAAGAACCTG 780
   TATCTGAAAT CCAAGAGGAA AAGCCTGAGC CAGTATTAGA AGAAACTGCC CCTGAGGATG 840
   CTCAGAAGAG TTCTTCTCCA GCACCTGCAG ACATAGCTCA GACAGTACAG GAAGACTTGA 900
   GGACATTTTC TTGGGCATCT GTGACCAGTA AGAATCTTCC ACCCAGTGGA GCTGTTCCAG 960
   TTACTGGGAT ACCACCTCAT GTTGTTAAAG TACCAGCTTC ACAGCCCCGT CCAGAGTCTA 1020
   AGCCTGAATC TCAGATTCCA CCACAAAGAC CTCAGCGGGA TCAAAGAGTG CGAGAACAAC 1080
   GAATAAATAT TCCTCCCCAA AGGGGACCCA GACCAATCCG TGAGGCTGGT GAGCAAGGTG 1140
   ACATTGAACC CCGAAGAATG GTGAGACACC CTGACAGTCA CCAACTCTTC ATTGGCAACC 1200
   TGCCTCATGA AGTGGACAAA TCAGAGCTTA AAGATTTCTT TCAAAGTTAT GGAAACGTGG 1260
   TGGAGTTGCG CATTAACAGT GGTGGGAAAT TACCCAATTT TGGTTTTGTT GTGTTTGATG   1320
   ATTCTGAGCC TGTTCAGAAA GTCCTTAGCA ACAGGCCCAT CATGTTCAGA GGTGAGGTCC   1380
   GTCTGAATGT CGAAGAGAAG AAGACTCGAG CTGCCAGGGA AGGCGACCGA CGAGATAATC   1440
   GCCTTCGGGG ACCTGGAGGC CCTCGAGGTG GGCTGGGTGG TGGAATGAGA GGCCCTCCCC   1500
   GTGGAGGCAT GGTGCAGAAA CCAGGATTTG GAGTGGGAAG GGGGCTTGCG CCACGGCAGT   1560
   AATCTTCATG GATCTTCATG CAGCCATACA AACCCTGGTT CCAACAGAAT GGTGAATTTT   1620
   CGACAGCCTT TGGTATCTTG GAGTATGACC CCAGTCTGTT ATAAACTGCT TAAGTTTGTA   1680
   TAATTTTACT TTTTTTGTGT GTTAATGGTG TGTGCTCCCT CTCCCTCTCT TCCCTTTCCT   1740
   GACCTTTAGT CTTTCACTTC CAATTTTGTG GAATGATATT TTAGGAATAA CGGACTTTTA   1800
   CCCGAATTCG TAATCATGGT CATAGCTGTT TCCGTGTGAA ATTGTTATCC GCTCACAATT   1860
   CCACACAACA TACGAGCCGG AAGCATAAAG TGTAAAGCCT GGGGTGCCTA ATGAGTGAGC   1920
   TAACTCACAT TAATTGCGTT GCGCTCACTG CCCGCTTTCC AGTCGGGAAA CCTGTCGTGC   1980
   CAGCGCATTA ATGAATCGGC CAACGCGCGG GGAGAGGCGG TTTGCGTATT GGGCGCCAGG   2040
   GTGGTTTTCT TTTCACCAGT GAGACGGGCA ACAGCTGATT GCCCTTCACC GCTGGCCCTG   2100
   AGAGAGTTGC AGCAAGCGGT CCACGCTGG                                     2129